# EUROPEAN PATENT SPECIFICATION

(11) **EP 1 192 955 B1**
(45) Date of publication and mention of the grant of the patent: **13.08.2008**
(21) Application number: 01308192.2
(22) Date of filing: 26.09.2001
(51) Int. Cl.: A61L 15/20, A61L 15/34

(54) **Absorbent article and barrier agent for absorbent article**
Saugfähiger Einwegartikel und Schutzmittel
Article absorbant et agent de barrière pour article absorbant

(30) Priority: 28.09.2000 JP 2000297140
(43) Date of publication of application: 03.04.2002
(73) Proprietor: UNI-CHARM CORPORATION, Shikokuchuo-shi, Ehime-ken (JP)
(72) Inventor: Hisanaka, Takayuki, c/o Technical Center, Mitoyo-gun, Kanagawa-ken 769-1602 (JP); Miyazawa, Kiyoshi, c/o Technical Center, Mitoyo-gun, Kanagawa-ken 769-1602 (JP); Endo, Tomoko, c/o Technical Center, Mitoyo-gun, Kanagawa-ken 769-1602 (JP)
(74) Representative: Fitchett, Stuart Paul

(56) References cited:
- WO-A-01/34086
- WO-A-99/56796
- IE-B- 32 438
- US-A- 5 593 682
- US-A- 5 609 587
- DATABASE WPI Section Ch, Week 200035 Derwent Publications Ltd., London, GB; Class B02, AN 2000-402881 XP002209234 & JP 2000 110067 A (DAIWABO CO LTD), 18 April 2000 (2000-04-18)
- DATABASE WPI Section Ch, Week 200027 Derwent Publications Ltd., London, GB; Class A25, AN 2000-313018 XP002209235 & JP 2000 095838 A (BRIDGESTONE CORP), 4 April 2000 (2000-04-04)
- DATABASE WPI Section Ch, Week 199736 Derwent Publications Ltd., London, GB; Class B05, AN 1997-389337 XP002209236 & JP 09 169638 A (SEKISUI CHEM IND CO LTD) , 30 June 1997 (1997-06-30)
- DATABASE WPI Section Ch, Week 198818 Derwent Publications Ltd., London, GB; Class D22, AN 1988-126486 XP002209237 & ZA 8 705 106 A (SMALBERGER J_), 13 January 1988 (1988-01-13)

## Description

### TECHNICAL FIELD

The present invention relates to an absorbent article such as a diaper, sanitary napkin, or the like which can reduce dermatitis (rash) brought on when such an absorbent article is worn, and to a barrier agent to be applied to the surface of the absorbent article. More specifically, the present invention relates to an absorbent article such as a diaper, sanitary napkin, or the like which can prevent contact-type dermatitis (rash) from being produced due to chemical stimulation from body fluids such as feces, urine, menstrual bleeding, and the like or due to physical stimulation of the skin by friction with respect to the surface materials of the absorbent article when such an absorbent article is worn, and to a barrier agent to be applied to the surface of the absorbent article for the said purpose.

### BACKGROUND ART

A problem with an absorbent article such as a diaper, sanitary napkin, or the like is dermatitis produced by attachment of feces, urine, menstrual bleeding, and the like to the skin, or stimulation of the skin by chemicals such as a surfactant used for improving the surface properties of the absorbent article, or contact-type dermatitis produced by a physical stimulation of the skin due to friction between the skin and the surface material of the absorbent article.

In addition, a synergistic action of these chemical and physical stimulators increases stimulation of the skin and swells the skin, resulting in an increase in the coefficient of friction between the surface material of the absorbent article and the skin.

In order to prevent such contact dermatitis (rash), it is important to thoroughly wipe off body fluids attached to the skin when an absorbent article is replaced, thereby maintaining the skin clean. However, it is impossible to completely avoid contact of body fluids with the skin which may occur during the period after a body fluid has become attached to the skin and before the absorbent article is replaced. The effort of frequently replacing the absorbent article can shorten the period of time during which the body fluids are in contact with the skin. However, there is a limitation to the number of times of replacement.

One method for preventing contact dermatitis is to apply a cream, oil, or the like to the skin of the wearer such as an infant. Although it is possible to apply a cream, oil, or the like to the buttocks or the anal region of the wearer when the diaper is replaced, application of such an agent each time the diaper is replaced is not only troublesome, but also may create a problem associated with sanitation. That is, the diaper wearer who has weak skin such as an infant or an aged person wearer may be infected with bacteria via their fingers of the care person or helper who applies the cream to the diaper wearer.

It has been tried to apply a skin care substance to the surface of absorbent articles in order to solve these problems. As one of these trials Japanese Patent Application Laid-open No. 1265/1990 discloses a method of using a pH-adjusting agent with an absorbent article for incontinence. This method has been developed based on the finding that the cause of diaper rash is ammonia produced by bacteria from feces or by the contact of an enzyme with urine. The ammonia increases the pH of the skin and causes a rash to occur. However, a rash caused by a substance other than ammonia cannot be prevented by a pH-adjusting agent.

Japanese Patent Applications Laid-open No. 509895/1998, No. 509896/1998, No. 510082/1999, and No. 510416/1999 disclose disposable absorbent articles with a specific lotion applied to the surface. The lotion comprises an emollient agent for improving lubricity and an immobilizing agent to attach the emollient agent to the surface of the absorbent articles. The emollient agent in these absorbent articles melts by the wearer's body temperature and becomes attached to the skin. The emollient agent transferred on the skin surface is expected to reduce stimulation of the skin by body fluids and protect the skin from physical stimulation by increasing lubricity between the surface of the absorbent article and the skin.

However, experiments by the present inventors have revealed the absorbent articles disclosed by Japanese Patent Applications Laid-open No. 509895/1998, No. 509896/1998, No. 510082/1999, and No. 510416/1999 cannot sufficiently prevent a rash in the diaper wearers.
- Document WO 99/56796 describes an absorbent article and the barrier agent for the absorbent article comprising vitamin E, an emollient and an paraffin wax as an immobilizing agent.
- Documents US-A-5 609587, JP 2000 110067 A, JP 2000 095838 A and WO 01/34086 separately describes an absorbent article and the barrier agent for the absorbent article comprising an oil soluble antioxidant, an emollient and an immobilizing agent.
- Document US-A-5 593 682 describes an skin treatment composition to use as topical application for reducing diaper rash comprising d-a-tocopheryl acetate, hydrogenated coconut oil and a glycerol ester of fatty acid.

### SUMMARY OF THE INVENTION

An object of the present invention is therefore to solve the above problems in those prior arts. Specifically, an object of the present invention is to provide an absorbent article such as a diaper, sanitary napkin, or the like which can prevent a rash in the wearer, or to provide a barrier agent to be applied to the surface of such an absorbent article.

More specifically, an object of the present invention is to provide a barrier agent to be applied to the surface of an absorbent article, which can prevent contact-type dermatitis (rash) due to chemical stimulation by body fluids, surfactants, etc. or physical stimulation of the skin by friction with the surface of the absorbent article, or to provide an absorbent article to which the barrier agent has been applied.

As a result of extensive studies for attaining the above object, the present inventors have found that, in addition to chemical stimulation and physical stimulation by the above body fluids, amino acids and sebum which are spodogenous materials of body fluids remaining on the skin are a cause of contact-type dermatitis (rash). This finding has led to the completion of the present invention. More specifically, the present inventors have found that in the absorbent articles disclosed by Japanese Patent Applications Laid-open No. 509895/1998, No. 509896/1998, No. 510082/1999, and No. 510416/1999, spedogenous materials or body fluids attached to the skin are covered by the lotion composition, then easily deteriorate into substances which stimulate the skin and induce rash. The present inventors have conceived ah idea of adding an anti oxidant to the barrier composition to prevent the spodogenous materials from being oxidized and deteriorating and completed the present invention.

Therefore, the present invention relates to:

A barrier agent for an absorbent article comprising an oil soluble antioxidant from the vitamin E group, coconut oil fatty acid triglyceride as emollient and an immobilizing agent selected from the group consisting of a glycerol ester of fatty acid, paraffin wax, and a higher alcohol.

An absorbent article comprising a surface to which a barrier agent described above has been applied.

Application of the barrier agent having antioxidant of the present invention to the surface of an absorbent article brings about the effect of preventing generation of an oxidative odor or oxidation of oils and fats, and preventing a skin rash even after wearing the absorbent article for many hours.

### DETAILED DESCRIPTION OF THE INVENTION

The present invention will be explained in more detail in the following description.

The absorbent article in the present invention includes disposal diapers, sanitary napkins, vaginal discharge liners, incontinence shorts, training pants, diaper holders, and the like.

The barrier agent of the present invention may be applied to the contacting area of these absorbent articles with the skin, for example, the top sheet, the three-dimensional gathers, the side flap, the waist gathers, and so on. Application to the top sheet to which feces or urine is frequently attached is particularly preferable.

An oil soluble antioxidant which is mutually soluble with an oily emollient agent is preferable as the antioxidant. As compounds which are oil soluble and have an antioxidant function, vitamins from the vitamin E group, are used.

These antioxidant agents can be used either individually or in combination of two or more. Vitamins are prefered due to the high degree of safety with respect to human body and few side effects. In addition, vitamins are widely used in articles coming directly into contact with the skin, such as cosmetics, and are known to exhibit the required effect when directly attached to skin.

vitamins in the present invention include vitamins and derivatives thereof.

Vitamin E group of the present invention includes vitamin E (tocopherol), isomers and derivatives thereof such as DL-α-tocopherol, DL-α-tocopherol acetate, DL-α-tocopherol succinate, calcium DL-α-tocopherol succinate, and the like. Of these, vitamin E is particularly preferable due to the high antioxidation effect.

The emollient agent in the present invention refers to a lubricity promoting substance that has a function to soften, relax or coat the skin, and to make the skin flexible, smooth, moisturized, and clean. An oily substance which is liquid or semi-solid at ambient temperature (25°C) is preferable as an emollient agent.

Although emollient agents are known (see Japanese Patent Applications Laid-open No. 509895/1998, No. 509696/1998, No. 510082/1999, and No. 510416/1999, for example).

The preferred emollient is coconut oil fatty acid triglyceride. Coconut oil fatty acid triglyceride is highly safe with respect to human body and exhibits almost no side effects. In addition, triglycerides are compounds constituting sebum, which is known to exhibit an emollient effect on the human skin surface. Therefore, selection of a triglyceride is particularly preferable.

The immobilizing agent is a substance for fixing and immobilizing the emollient agent on the surface of an absorbent article, is soluble or dispersible in oils or fats, and, if mixed with an emollient agent, increases the viscosity of the emollient agent, making the emollient agent easily applied to the surface of the absorbent article. The immobilizing agent preferably has a melting point of 40-90°C and a surface tension of 25-45×10⁻³Nm (25-45 dyne/cm²) at 20°C.

Immobilizing agents are disclosed in the aforementioned Japanese Patent Applications Laid-open No. 509895/1998, No. 509896/1998, No. 510082/1999, and No. 510416/1999. Preferable immobilizing agents are oils and fats of animal or vegetable origin, fatty acid esters,paraffin wax, and higher alcohols, which are highly safe for human body and exhibit almost no side effects. Particularly preferable compounds are fatty acid glycerides, esters of C₁₂₋₂₂ fatty acid and dextrin, paraffin wax, and higher alcohols having a melting point of 40-90°C.

As the immobilizing agent or the emollient agent, as described above, oils and fats of animal or vegetable origin can be used.

For the reason, preferably raw fats and oils are hydrolyzed to fatty acids and glycerol, and the fatty acids obtained are separated and purified to desirable fatty acids by distillation. The fatty acids and glycerol obtained are composed to glycerol ester of fatty acid again. Said glycerol ester of fatty acid has properties controlled suitable for the immobilizing agent or the emollient agent, and thus is preferable for use to these agents.

Furthermore, these raw materials are eatable and have advantages that they are low risk and secure, even if the wearer of infant or aged person with senile dementia accidentally eats or laps the barrier agent applied

The fatty acid glyceride may be any one of a monoglyceride, diglyceride, and triglyceride, the number of carbon atoms of the fatty acid moiety thereof is preferably 2-35, and particularly preferably 8-22. Most preferable glycerides are the monoglyceride of stearic acid and the monoglyceride of lauric acid. Fatty acid dextrin esters include, for example, dextrin palmitate, dextrin stearate, dextrin behenate, dextrin myristate, dextrin coconut oil fatty acid ester, and dextrin laurate.

Paraffin wax having 16-40 carbon atoms, a melting point of 40-90°C, and a specific gravity at 20°C of 0.88-0.92 is preferable.

A higher alcohol having a carbon atom content of 14-32, particularly 16-22, is preferable. A particularly preferable higher alcohol is stearyl alcohol.

These immobilizing agents can be used either individually or in combination of two or more.

The barrier agent of the present invention comprises an antioxidant, an emollient agent and an immobilizing agent

In addition to the antioxidant, emollient agent and immobilizing agent, the barrier agent of the present invention may optionally comprise any components, which are commonly used for improving the performances of the absorbent articles. Such optional components may include an anti-inflammatory agent, pH-adjusting agent, and surfactant to improve absorbency. An antibacterial agent and humectant may also be added

The anti-inflammatory agent which can be used includes naturally occurring anti-inflammatory agents such as peony, ogon, hypericum, chamomile, glycyrrhiza, peach leaf, mugwort, and perilla frutescens extract, and synthetic anti-inflammatory agents such as allantoin and dipotassium glycyrrhizinate.

A pH-adjusting agent may be added to maintain the skin in a weakly acidic condition. Malic acid, succinic acid, citric acid, tartaric acid, lactic acid, and the like can be used as the pH-adjusting agent.

As a surfactant for improving absorbency, surfactants imparting a low irritation to the skin such as a sucrose fatty acid ester can be selected.

The barrier agent may be incorporated in the absorbent article in any manner which can permit the barrier agent to be present on the surface of the absorbent article,
(1) For example, the three component mixture comprising an emollient agent, antioxidant and immobilizing agent may be present as a single layer on the surface of the absorbent article. In this instance, the ratio of the total weight of the emollient agent and antioxidant to the weight of the immobilizing agent is 5-95:95-5, and preferably 30-70:70-30. The amount of the barrier agent per surface area of the absorbent is 0.5-50 g/m², and preferably 1-30 g/m². The amount of antioxidants is 0.1% or more of the barrier agent product, and preferably 0.5% or more.
(2) The barrier agent may be present on the surface of the absorbent article as a multi-layer.

In this instance, (i) a two-component mixture containing an emollient agent and immobilizing agent may form one layer on the absorbent article, then a three-component mixture containing an emollient agent, antioxidant and immobilizing agent may be present on the surface of that layer coming in direct contact with the skin. Alternatively, (ii) a two-component mixture containing an emollient agent and immobilizing agent may form one layer on the absorbent article, then another two-component mixture containing an antioxidant and immobilizing agent may be present on the surface of layer coming in direct contact with the skin.

Such a multi-layer structure allows a greater proportion of antioxidant to be present on the surface coming into contact with the skin so that the antioxidant may adhere to the skin before the emollient agent covers spodogenous materials attached to the skin, thereby increasing the effect of the present invention. In addition, because antioxidants are expensive materials, obtaining the target effect by using as small an amount of antioxidant as possible is desirable. In this respect, these methods of incorporating a larger amount of an antioxidant in the layer in contact with the skin are preferable.

In the above method (i) or (ii), the ratio of the emollient agent to immobilizing agent in the two-component mixture of the absorbent article side is 5-95:95-5, and preferably 30-70:70-30, and the amount of the mixture per surface area of absorbent article is 0.25-25 g/m², and preferably 0.5-15 g/m².

In the above method (i), the ratio of the total weight of the emollient agent and antioxidant to the weight of the immobilizing agent in the three-component mixture coming in contact with the skin is 5-95:95-5, and preferably 30-70:70-30, and the amount of the mixture per surface area of absorbent article is 0.25-25 g/m², and preferably 0.5-15 g/m².

In the above method (ii), the ratio of the antioxidant to immobilizing agent in the two-component mixture coming in contact with the skin is 5-95:95-5, and preferably 30-70:70-30, and the amount of mixture per surface area of absorbent article is 0.25-25 g/m², and preferably 0.5-15 g/m².

The application area of barrier agent per total area of the absorbent article in contact with the skin is 5-90%, and preferably 10-70%, either when the barrier agent is present in single layer as described in (1) above or present in multilayers as described in (2) above.

A common application method may be employed for applying the barrier agent to the surface material of absorbent article.

When applying the barrier agent to the liquid-permeable surface material, the area to which the barrier agent has been applied exhibits decreased permeability. In such a case, both a hydrophilic area and the applied area must be present on the sheet surface. This is preferably accomplished by patterning the area being applied the barrier agent, a gravure application method or flexography application method for the printing technique can be applicable therefor. In addition, an extrusion method, slot method, and spray method which are applications of a thermosensible adhesive application technique may also be used. A multi-layer can also be produced by these application methods.

The barrier agent of the present invention is applied to the surface of absorbent article coming in contact with the skin. Usually, this agent is applied to a top sheet. There are no specific limitations to the object to which the barrier agent is applied. Usually, such an object is a permeable sheet-like material such as a non-woven fabric or permeable porous film.

The non-woven fabric used in the absorbent article is made of 1-5 d fiber and has a density of 10-50 g/m². Fibers for non-woven fabric may be synthetic fiber such as polyolefin and polyester, semi-synthetic fiber such as rayon, or natural fiber such as cotton, pulp, and silk.

Permeable porous films usable in the present invention are made from thermoplastics by extrusion, followed by boring by heated needles, embossing, hot blast, or the like. Polyethylene (density: 0.86-1.1 g/cm³), polypropylene (density: 0.89-1.2 g/cm³), and the like can be used either individually or in combination (in single layer or multi-layer) as the thermoplastics for porous films.

A permeable sheet material must permit body fluids to permeate and must withstand 0-300 mm H₂O of water pressure according to the JIS L1092 (Test method for water resistance of textiles, water resistance test method A (low water pressure method)). In addition, the permeable sheet material must have gas permeability to permit water vapor to pass from the surface material to the absorbent in the range of 5-700 cm³/cm²/sec according to the JIS L1906 (Test methods for non-woven fabrics made of filament yarn, Frazier type permeameter test method).

The barrier agent of the present invention can be applied not only to absorbent articles, but also to skin care dry wipes such as a wound protection sheet, tissue paper, toilet paper and the like, or skin care wet wipes such as wet tissue and the like to prevent contact dermatitis (a rash) due to the use of these wipes. When applying to wet wipes, the barrier agent of the present invention is first applied to the surface of the wipes, followed by impregnation of an aqueous type drug solution. Wet wipes have both of an oily region and an aqueous region on the surface. The wet wipes not only can prevent inflammation due to an aqueous anti-inflammatory agent and remove aqueous soiled material by the aqueous region, but also can prevent contact dermatitis (a rash) due to the action of an oily barrier which is caused to adhere to the skin when the skin is wiped by the wet wipes.

### EXAMPLES

The present invention will be explained by way of examples in the following description, this is not intended to be limiting of the present invention.

### (Example 1)

A barrier agent was prepared by melting a mixture of coconut oil fatty acid triglyceride as an emollient agent, stearyl alcohol as an immobilizing agent, and vitamin E as an antioxidant at a weight ratio of 49.8:49.7:0.5. This barrier agent was applied to the surface material of a diaper in a stripe pattern in an amount of 20 g/m².

The diaper comprised a top sheet having high liquid permeability, an absorber made from pulp and a high water-absorptive resin capable of adsorbing and retaining body fluid, and a back sheet of polyethylene film. This was an assembling-type diaper to be fixed to the trunk using an adhesive tape. A 25 g/m² non-woven fabric to which polyolefin fiber had been melt-adhered was used as the top sheet. The previously prepared barrier agent described above was melted by heating and applied to the continuously supplied non-woven fabric for top sheet using a coater. After cooling, a diaper was manufactured by assembling the top sheet to which the barrier agent has been applied, the absorber and the back sheet.

The diaper was worn by a healthy infant having no erythema or the like in the external genital area. An odor of oxidized fats and oils, oxidation of fats and oils, and the state of the skin, one week after the diaper had first been worn, were observed according to the following method. As a result, no odor of oxidized fats and oils, no oxidation of fats and oils, and no change in the state of the skin were observed.

The following test methods were applied.

### [Odor of oxidized fats and oils]

Five hours after the infant had worn the diaper, the odor was sensorially determined.

### [Oxidation of fats and oils]

Five hours after the infant had worn the diaper, sebum on the skin surface was analyzed according to the following skin surface sebum analyzing method.
(i) 0.4 mg of sebum was collected from the skin surface in the diaper attached area using cotton impregnated a 1:1 mixture solvent of acetone and ether.
(ii) The sebum was extracted with ether and the extract was dried in a reduced pressure at 30°C or less with an aspirator while flowing nitrogen gas.
(iii) 0.4 mg of the dry material obtained in (ii) above was dissolved in 100 µl of 1-butanol. 10 µl of the solution was injected into a CL (chemiluminescence)-HPLC system. Operating conditions of the CL-HPLC system were as follows.
   Column: CAPCELL-PAK C18 (25 cm x 4.6 mm)
   Column temperature: 25°C
   Solvent: methanol
   Luminescence indication solvent: 10 µg/ml cytochrome C + 1.0 µg/ml borate buffer (pH=9.3)
   Flow rate: 1.1 ml/min.
   Detector: Chemiluminescence Analyzer
(iv) The fats and oils were judged to have been oxidized when a peak was observed as a result of the analysis.

### [State of the skin]

The diaper was replaced eight times every day. The infant wearing the diaper lived in a usual manner (eating, bathing, going outside, and so on). One week later, the diaper was removed and the state of the skin was immediately observed by the naked eye.

### (Example 2)

The experiment was carried out in the same manner as in Example 1, except for using a barrier agent consisting of a coconut oil fatty acid triglyceride, stearyl alcohol, and vitamin E at a weight ratio of 47.5:47.5:5.0. As a result, no odor of oxidized fats and oils, no oxidation of fats and oils, and no change in the state of the skin was observed.

### (Example 3)

The experiment was carried out in the same manner as in Example 1, except for using stearic acid monoglyceride as the immobilizing agent. As a result, no odor of oxidized fats and oils, no oxidation of fats and oils, and no change in the state of the skin were observed.

The stearic acid monoglyceride used here was a re-composed ester of glycerol and stearic acid. The stearic acid was obtained as stearic acid fraction by distillation of fatty acids that was separated from glycerol after hydrolyzing beef tallow.

### (Example 4)

The experiment was carried out in the same manner as in Example 2, except for using stearic acid monoglyceride as the immobilizing agent. As a result, no odor of oxidized fats and oils, no oxidation of fats and oils, and no change in the state of the skin were observed. The same stearic acid monoglyceride was used as in Example 3.

### (Example 5)

The experiment was carried out in the same manner as in Example 1, except for using lauric acid monoglyceride as the immobilizing agent. As a result, no odor of oxidized fats and oils, no oxidation of fats and oils, and no change in the state of the skin were observed.

The lauric acid monoglyceride used here was a re-composed ester of glycerol and lauric acid. The lauric acid was obtained as lauric acid fraction by distillation of fatty acids that was separated from glycerol after hydrolyzing beef tallow.

### (Example 6)

The experiment was carried out in the same manner as in Example 2, except for using lauric acid monoglyceride as the immobilizing agent. As a result, no odor of oxidized fats and oils, no oxidation of fats and oils, and no change in the state of the skin were observed. The same lauric acid monoglyceride was used as in Example 5.

### (Example 7)

The experiment was carried out in the same manner as in Example 2, except for using paraffin wax as the immobilizing agent. As a result, no odor of oxidized fats and oils, no oxidation of fats and oils, and no change in the state of the skin were observed.

### (Comparative Example 1)

The experiment was carried out in the same manner as in Example 1, except for using a barrier agent containing no antioxidant and containing coconut oil fatty acid triglyceride and stearyl alcohol at a weight ratio of 50.0:50.0. As a result, an odor of oxidized fats and oils was detected, oxidation of fats and oils was confirmed, and erythema was observed on the skin.

The results of Examples 1-7 and Comparative Example 1 are summarized in Tables 1 and 2.

**[Table 1]**

| | Emollient agent | Immobilizing agent | Antioxidant |
|---|---|---|---|
| Example 1 | Coconut oil fatty acid triglyceride (49.8) | Stearyl alcohol (49.7) | Vitamin E (0.5) |
| Example 2 | Coconut oil fatty acid triglyceride (47.5) | Stearyl alcohol (47.5) | Vitamin E (5.0) |
| Example 3 | Coconut oil fatty acid triglyceride (49.8) | Stearic acid monoglyceride (49.7) | Vitamin E (0.5) |
| Example 4 | Coconut oil fatty acid triglyceride (47.5) | Stearic acid Monoglyceride (47.5) | Vitamin E (5.0) |
| Example 5 | Coconut oil fatty acid triglyceride (49.8) | Lauric acid monoglyceride (49.7) | Vitamin E (0.5) |
| Example 6 | Coconut oil fatty acid triglyceride (47.5) | Lauric acid monoglyceride (47.5) | Vitamin E (5.0) |
| Example 7 | Coconut oil fatty acid triglyceride (47.5) | Paraffin wax (47.5) | Vitamin E (5.0) |
| Comparative Example 1 | Coconut oil fatty acid triglyceride (50.0) | Stearyl alcohol (50.0) | Vitamin E (0.0) |

**[Table 2]**

| | 2) Odor of oxidized fats and oils | 3) Oxidation of fats and oils (CL-HPLC peaks) | 4) State of the skin |
|---|---|---|---|
| Example 1 | No odor | No peaks | No change |
| Example 2 | No odor | No peaks | No change |
| Example 3 | No odor | No peaks | No change |
| Example 4 | No odor | No peaks | No change |
| Example 5 | No odor | No peaks | No change |
| Example 6 | No odor | No peaks | No change |
| Example 7 | No odor | No peaks | No change |
| Comparative Example 1 | Odor was sensed | Peaks appeared | Slight erythema was observed |

It can be seen from the results shown in Tables 1 and 2 that the oxidation of fats and oils can be inhibited in an absorbent article applied the barrier agent by adding antioxidant into the barrier agent, and change in the state of the skin of wearer can be avoided. Though beef tallow was used as raw material of stearic acid monoglyceride and lauric acid monoglyceride in Examples 3-6, such a raw material should not be limited to beef tallow and other raw materials such as coconut oil or the like may be used and obtained same effects.

### INDUSTRIAL APPLICABILITY

Application of the barrier agent of the present invention to the surface of an absorbent article brings about the effect of preventing generation of an oxidative odor or oxidation of oils and fats, and preventing a skin rash even after wearing the absorbent article for many hours. Therefore, the present invention is very useful as an absorbent article such as disposal diapers, sanitary napkins, vaginal discharge liners, incontinence shorts, training pants, diaper holders, and the like, and also useful as a barrier agent for applying on the surface thereof.

## Claims

1. A barrier agent for an absorbent article comprising an oil soluble antioxidant from the vitamin E group, coconut oil fatty acid triglyceride as emollient and an immobilising agent selected from the group consisting of glycerol ester of a fatty acid, paraffin wax and a higher alcohol.

2. An absorbent article comprising a surface to which a barrier agent according to Claim 1 has been applied.

## Patentansprüche

1. Ein Sperrmittel für einen absorbierenden Artikel, umfassend ein öllösliches Antioxidans aus der Vitamin-E-Gruppe, Kokosnussöl-Fettsäure-Triglycercid als Weichmacher sowie ein Immobilisierungsmittel aus einer Gruppe bestehend aus einem Fettsäure-Glycerolester, Paraffinwachs und einem höheren Alkohol.

2. Ein absorbierender Artikel mit einer Oberfläche, auf die ein Sperrmittel gemäß Anspruch 1 aufgebracht wurde.

## Revendications

1. Agent barrière pour un article absorbant comprenant un antioxydant soluble dans l'huile issu du groupe de vitamines E, du triglycéride d'acide gras d'huile de noix de coco comme émollient et un agent d'immobilisation sélectionné parmi le groupe comprenant un ester de glycérol d'un acide gras, de la paraffine et un alcool plus élevé.

2. Article absorbant comprenant une surface sur laquelle un agent barrière selon la revendication 1 a été appliqué.
